# FASCICULE DE BREVET EUROPEEN

(11) **EP 3 134 100 B1**
(45) Date de publication et mention de la délivrance du brevet: **11.12.2019**
(21) Numéro de dépôt: 15715310.7
(22) Date de dépôt: 16.04.2015
(51) Int. Cl.: A61K 36/36, A61Q 19/00, A61K 8/97, A61K 8/34, A61K 8/04, A61Q 19/08

(54) **COMPOSITIONS COSMETIQUES A APPLICATION TOPIQUE COMPRENANT DES CELLULES VEGETALES DE BOUGAINVILLIER**
KOSMETISCHE ZUSAMMENSETZUNGEN ZUR TOPISCHEN ANWENDUNG MIT BOUGAINVILLEA-PFLANZENZELLEN
COSMETIC COMPOSITIONS FOR TOPICAL APPLICATION COMPRISING BOUGAINVILLEA PLANT CELLS

(30) Priorité: 23.04.2014 FR 1453646
(43) Date de publication de la demande: 01.03.2017
(73) Titulaire: Société De Recherche Cosmétique S.à.r.L., 2314 Luxembourg (LU)
(72) Inventeur: LECLERE, Jacques, F-45500 Saint-gondon (FR); ENNAMANY, Rachid, F-33000 Bordeaux (FR)
(74) Mandataire: Peguet, Wilfried
(86) Numéro de dépôt international: PCT/EP2015/058240
(87) Numéro de publication internationale: WO 2015/162051

(56) Documents cités:
- EP-A2- 1 985 280
- WO-A2-2013/102882
- FR-A1- 2 994 843
- JP-A- S63 192 705
- CORNELIA SCHÜRCH ET AL: "Potential of plant cells in culture for cosmetic application", PHYTOCHEMISTRY REVIEWS, vol. 7, no. 3, 1 décembre 2007 (2007-12-01), pages 599-605, XP055043814, ISSN: 1568-7767, DOI: 10.1007/s11101-007-9082-0
- DATABASE WPI Week 200730 1 janvier 2007 (2007-01-01) Thomson Scientific, London, GB; AN 2007-305799 XP002733987, & JP 2007 084448 A (SHISEIDO CO LTD) 5 avril 2007 (2007-04-05)

## Description

La présente invention se rapporte au domaine technique général des produits cosmétiques à usage topique, utilisables notamment sur la peau.

Plus précisément, la présente invention est relative l'utilisation de cellules végétales dédifférenciées et élicitées de Bougainvillier à titre d'ingrédient actif dans une composition cosmétique. Elle se rapporte également aux compositions cosmétiques à application topique comprenant de telles cellules, à un procédé cosmétique non thérapeutique de traitement de la peau mettant en œuvre une telle composition cosmétique, ainsi qu'à l'utilisation non thérapeutique d'une composition cosmétique à application topique comprenant de telles cellules pour le soin de la peau, en particulier pour améliorer la fermeté de la peau, notamment en favorisant la régénération du derme et de l'épiderme de la peau.

La peau est un véritable organe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement.

L'épiderme, principalement composé de kératinocytes, de mélanocytes et des cellules de Langerhans, joue un rôle fondamental pour assurer la protection et le maintien d'une bonne trophicité. Les kératinocytes sont des cellules extrêmement dynamiques qui subissent une prolifération et une différenciation permanentes aux termes desquelles elles se transforment en cellules mortes (cornéocytes), s'éliminant régulièrement par desquamation.

Le derme se compose principalement de collagène, d'élastine et de protéoglycanes, molécules synthétisées par les fibroblastes dermiques. Les fibres de collagène assurent la résistance mécanique et la texture de la peau, l'élastine est responsable de l'élasticité, et les protéoglycanes, combinant protéine et glycosaminoglycane, jouent un rôle majeur de structure et d'hydratation de la peau. Les glycosaminoglycanes, tels que l'acide hyaluronique, sont des molécules très hygroscopiques qui sont capables de retenir des quantités importantes d'eau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

La peau est en renouvellement constant. La desquamation des cellules en surface est compensée par la naissance de nouvelles unités en profondeur. Lorsque les kératinocytes ont perdu leur noyau et leurs organites cellulaires, ils prennent le nom de cornéocytes. Avec les frottements, ils sont arrachés ou se décrochent sous l'action programmée d'enzymes spécifiques : c'est le phénomène de desquamation.

Les agressions de l'environnement, y compris les agressions climatiques telle que l'exposition au soleil, les variations de températures, la pollution, le stress, la fatigue, et le vieillissement en général ont tendance à altérer les capacités naturelles de régénération des kératinocytes composant l'épiderme qui devient alors moins dense, entrainant l'apparition de signes de vieillissement cutanés tels que rides et ridules et une fermeté diminuée.

Pour atténuer ou éliminer les signes du vieillissement de la peau, on a proposé divers traitements à base de compositions telles que crèmes et lotions contenant des alpha-hydroxyacides ou des rétinoïdes, appliquées régulièrement pour réduire progressivement le nombre de rides et ridules. On a aussi proposé des implants de collagène pour dissimuler les lignes d'expression autour des yeux ou de la bouche, la dermabrasion et les peelings chimiques pour éliminer la couche supérieure de la peau endommagée, la chirurgie esthétique, telle que la blépharoplastie (chirurgie des paupières) ou un lifting pour rajeunir une peau présentant un affaissement, ou encore une restructuration à l'aide d'un laser au dioxyde de carbone pour éliminer les ridules. Le brevet FR-A-2.783.169 décrit l'utilisation de pentapeptides du type Lys-Thr-Thr-Lys-Ser dans des compositions topiques pour favoriser la synthèse du collagène et des glycosaminoglycanes, et par conséquent la régénération cutanée.

Toutefois, si tous ces produits et méthodes connus peuvent avoir un effet favorable sur les signes du vieillissement cutané, par exemple en masquant ou en réduisant les rides, ils sont généralement sans incidence sur l'évolution cellulaire qui aboutit à ces signes du vieillissement.

Les produits d'origine naturelle ou contenant des composés naturels sont par ailleurs de plus en plus appréciés par les consommateurs et consommatrices de produits cosmétiques. C'est pourquoi on trouve sur le marché des produits cosmétiques de plus en plus de produits contenant des substances naturelles ou d'origine naturelle.

La présente invention vise à fournir notamment une composition cosmétique permettant de lutter efficacement contre les signes du vieillissement de la peau, et plus particulièrement permettant de régénérer la peau telle que le derme et l'épiderme.

Ainsi, la présente invention a pour premier objet l'utilisation de cellules végétales dédifférenciées et élicitées de Bougainvillier à titre d'ingrédient actif dans une composition cosmétique.

En effet, les études effectuées par la société Demanderesse ont montré que des cellules dédifférenciées et élicitées de culture végétale de Bougainvillier, lorsqu'elles sont appliquées sur la peau, sont incorporées dans les différentes couches de l'épiderme et ont un effet positif sur la prolifération des cellules de l'épiderme, favorisant ainsi sa régénération. De plus, aux doses utilisées, les essais effectués ont montré que les compositions cosmétiques à base de cellules dédifférenciées et élicitées de Bougainvillier utilisée dans la présente invention ne présentent aucune cytotoxicité.

Le Bougainvillier est un genre d'arbuste de la famille des *Nyctaginaceae.* Certaines espèces sont appelées bougainvillée (féminin) ou bougainvillier, (masculin) notamment *Bougainvillea glabra*, *Bougainvillea spectabilis* et *Bougainvillea buttiana.* Le premier spécimen de l'une de ces espèces a été découvert par le botaniste Philibert Commerson au brésil, lors d'une expédition dirigée par l'explorateur français Louis Antoine de Bougainville dont il tire son nom. Les bougainvilliers sont des arbustes épineux grimpants aux vives couleurs qui contrairement aux apparences ne sont pas dues aux fleurs. Celles-ci sont petites et blanches, et ce sont les bractées de l'extrémité des rameaux qui entourent qui offrent des coloris variés rose, rouge, mauve, orange, jaune ou blanc. Ces arbustes sont largement utilisés comme plantes ornementales dans les régions tempérées chaudes, mais à ce jour, on ne lui connait pas d'application en cosmétique.

Toutes les espèces de Bougainvillier peuvent être utilisées selon l'invention, mais on peut plus particulièrement mentionner *Bougainvillea glabra*, *Bougainvillea alba, Bougainvillea spectabilis*, *Bougainvillea infesta,* et *Bougainvillea buttiana.*

Par cellules végétales dédifférenciées de Bougainvillier, on entend toute cellule végétale issue d'une culture de cellules de Bougainvillier, lesdites cellules ne présentant aucun des caractères d'une spécialisation particulière et étant capables de vivre par elles-mêmes et non en dépendance avec d'autres cellules.

Les cellules dédifférenciées et élicitées de Bougainvillier utilisables selon la présente invention peuvent être obtenues par des techniques connues de croissance ou multiplication cellulaire, en milieu liquide ou solide, de cellules de Bougainvillier cultivées *in vitro* dans un environnement contrôlé, dans des conditions aseptiques en l'absence de micro-organismes telles que par exemple selon les méthodes décrites par E. F. George et P. D. Sherrington dans Plant Propagation by tissue culture, Handbook and Directory of Commercial Laboratories (Exegetics Ltd, 1984).

La technique de la culture végétale présente l'avantage de produire des cellules cultivées dans des conditions environnementales bien définies et reproductibles, en ce qui concerne notamment la température, la lumière et le milieu de culture, et de produire des cellules non différenciées se trouvant au même stade physiologique à un instant donné. Un autre avantage est la formation de métabolites secondaires en un temps plus court, de l'ordre de une à trois semaines, que chez la plante où ce délai est de plusieurs mois.

Par exemple, on peut préparer des cellules dédifférenciées de Bougainvillier à partir d'explants de feuilles, de fleurs, de la tige ou de la racine.

Les milieux de culture utilisables selon l'invention sont ceux généralement connus de l'homme du métier. On peut notamment citer à titre d'exemple les milieux de Gamborg, de Murashige et Skood, de Heller, de White, etc ... et dont la description complète peut être trouvée dans « Plant Culture Media : formulations and uses », E. F. George, DJM Puttock et H. J. George (Exegetics Ltd 1987, Tomes 1 & 2).

On entend par « élicitation », la mise en œuvre d'un stress ou d'une agression (biologique, chimique ou physique) sur les cellules végétales dans leur milieu de culture afin de provoquer un ou plusieurs mécanismes de défense se concrétisant notamment, mais non uniquement, par la synthèse de métabolites secondaires d'intérêt tels que les phytoalexines.

Selon l'invention, l'élicitation des cellules dédifférenciées de Bougainvillier en milieu de culture peut être réalisée par application d'agents chimiques ou de stresses divers tels que pression, dépression, vide, variation de pression, présence d'un gaz, atmosphère variable, chaleur, froid, lumière, cycles de luminosité, radiations, toxines, agitation, contamination par des microorganismes (bactéries, levures, virus), ultrasons, rayonnement infra rouge ou ultraviolet, asphyxie, etc ...

Suivant la présente invention, on peut plus particulièrement utiliser des cellules dédifférenciées, élicitées et lyophilisées de Bougainvilliers. La lyophilisation des cellules permet d'améliorer leur conservation et, par suite, de faciliter leur incorporation dans les compositions cosmétiques.

Les éléments dosés dans les cellules lyophilisées sont les suivants (pour 100 g de cellules lyophilisées) :
**Protéines** (déterminée selon la méthode décrite dans l'Arrêté du 8 septembre 1977 relatif aux méthodes officielles d'analyse des produits diététiques et de régime) : 13,56 g

L'aminogramme de l'extrait protéique a mis en évidence les principaux acides aminés suivants. Dans ce qui suit, les teneurs en acides aminés sont indiquées en grammes pour 100 g de cellules lyophilisées.

| | |
|---|---|
| Acide aspartique | 10,61 |
| Acide glutamique | 10,15 |
| Proline | 6,75 |
| Lysine | 6,38 |
| Valine | 5,79 |
| Alanine | 5,29 |
| Sérine | 5,27 |
| Glycine | 4,82 |
| Arginine | 4,72 |
| Glutamine | 4,63 |
| Isoleucine | 4,61 |
| Leucine | 4,60 |
| Phénylalanine | 4,25 |
| Thréonine | 4,01 |
| Tyrosine | 3,70 |
| Asparagine | 3,29 |
| Tryptophane | 3,02 |
| Méthionine | 2,58 |
| Cystéine | 1,89 |
| Ornithine | 0,24 |

**Lipides** (déterminés selon la méthode décrite dans l'Arrêté du 8 septembre 1977 relatif aux méthodes officielles d'analyse des produits diététiques et de régime) : 1,08 g, se composant notamment de 73 % en masse environ d'acides gras saturés et insaturés en C₁₈ et de 21 % en masse environ d'acides gras saturés en C₁₆.

**Sucres** (déterminés par Chromatographie ionique - Détection ampérométrique pulsée (PAD)) :

| | |
|---|---|
| - Glucose | 11,23 g |
| - Fructose | 13,10 g |
| - Lactose | < 0,12 g |
| - Saccharose | 10,78 g |
| - Maltose | < 0,13 g |

Selon une forme de réalisation particulièrement avantageuse de l'invention, les cellules dédifférenciées et élicitées de Bougainvillier sont utilisées sous la forme d'une suspension dans la glycérine. De préférence, la quantité de cellules dédifférenciées et élicitées de Bougainvillier au sein de ladite suspension varie de 20 à 30 % en masse environ, et encore plus préférentiellement est de l'ordre de 25 % en masse environ par rapport à la masse totale de ladite suspension.

L'invention a aussi pour objet une composition cosmétique à application topique caractérisée en ce qu'elle comprend, à titre d'ingrédient actif, des cellules végétales dédifférenciées et élicitées de Bougainvilliers et au moins un support cosmétiquement acceptable.

De façon avantageuse, la composition cosmétique conforme à l'invention comprend de 0,001 à 10 % en masse d'une suspension de cellules végétales dédifférenciées et élicitées de Bougainvillier telle que décrite ci-dessus, par rapport à la masse totale de la composition cosmétique, et encore plus préférentiellement de 0,1 à 5 % en masse.

Au sens de la présente invention, on entend par « support cosmétiquement acceptable », les milieux comprenant de l'eau, un mélange d'eau et d'un ou plusieurs solvants organiques, ou un solvant ou un mélange de solvants organiques cosmétiquement acceptables.

Les compositions cosmétiques selon l'invention peuvent contenir, outre les cellules végétales dédifférenciées et élicitées de Bougainvillier, un ou plusieurs ingrédient actifs secondaires renforçant ou complétant avantageusement leur activité, et compatibles, c'est-à-dire non susceptibles de réagir les uns avec les autres ou de masquer ou limiter leurs effets.

Plus particulièrement, les actifs secondaires peuvent être choisis parmi un agent anti-âge, ou parmi l'acétyl tetrapeptide-5 (CAS N° 820959-17-9), un extrait de plancton, un extrait de maca qui a un effet sur la prévention de dérèglements liés à une déficience de la microcirculation cutanée et au stress oxydatif qui peut en résulter, un extrait de pétales de coquelicot agissant sur la nutrition cellulaire, une combinaison d'extraits d'anchuse, de coquelicot et de passiflore qui, en association, ont un effet dans la prévention des signes du vieillissement cutané, un extrait de graines de mimosa, un extrait de pétales de souci, un extrait de barbitamao, un extrait de kigelia africana, un extrait d'avoine, la vitamine C, la vitamine E et l'hexylrésorcinol.

L'agent anti-âge peut être par exemple un lipide tel que le géranyl géranyl isopropanol (Juvinity®) qui réduit la formation des peroxydes intracellulaires et retarde ainsi le vieillissement cellulaire.

Les compositions cosmétiques conformes à la présente invention peuvent se présenter sous les formes galéniques classiquement utilisées pour une application topique, c'est-à-dire sous forme de gel, d'émulsion (en particulier crème ou lait), d'émulsion biphasique huile-dans-eau ou eau-dans-huile, d'huile corporelle, de masque, de pommade, d'onguent, de lotion, de solution concentrée, lesdites formes galéniques contenant en outre des excipients et supports usuels et acceptables sur le plan cosmétologique. Elles sont utilisées de préférence sous forme de crèmes, de sérums, de lotion ou de gel.

Ces formes d'administration par voie topique sont préparées par les techniques usuelles, et par exemple, dans le cas d'une crème, par dispersion d'une phase grasse dans une phase aqueuse pour obtenir une émulsion huile-dans-eau, ou inversement pour préparer une émulsion eau-dans-huile. Dans le cas de crèmes, on peut utiliser des émulsions à structure lamellaire obtenues avec des lécithines hydrogénées ou des sucro-esters, contenant peu de produits éthoxylés ou n'en contenant pas du tout.

Les compositions topiques selon l'invention peuvent comprendre des excipients appropriés pour une application topique externe, en particulier des excipients acceptables sur le plan cosmétologique. Ces excipients appropriés pour la formulation sont bien connus de l'homme du métier et comprennent en particulier des agents de pénétration tels que le phytantriol, l'octyl dodécanol et l'escine ; les épaississants tels que les gommes naturelles et les polymères de synthèse ; les tensioactifs ; les émollients tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels que des dérivés de polyglycérol ; les conservateurs tels que le phénoxyéthanol et l'acide déhydroacétique ; les colorants ; les parfums ; etc...

Comme autres ingrédients utilisables dans les compositions de l'invention, on peut citer les agents hydratants tels que la glycérine, le butylène glycol, le sel de sodium de l'acide pyrrolidone carboxylique (sodium PCA), ainsi que des associations de dérivés glucosiques à effet hydratant et restructurant comme le produit Aquaxyl® (xylitylglucoside et anhydroxylytol et xylitol), et également les vitamines antioxydantes telles que la vitamine E, par exemple l'acétate de tocophérol ou le tocotriénol, la vitamine C, les polyphénols naturels.

On peut aussi ajouter à la composition des glucides choisis principalement parmi le glucose, le glycogène et le tréhalose, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

On peut également ajouter à la composition des agents de protection contre les rayons ultraviolets, et par exemple des filtres solaires UV-A et UV-B hydrophiles ou lipophiles, ou des pigments formant écran anti-ultraviolet.

Les filtres solaires peuvent être choisis par exemple parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane.

Les pigments peuvent être choisis par exemple parmi le dioxyde de titane, l'oxyde de zinc et l'oxyde de zirconium.

Les compositions cosmétiques à application topique de l'invention ont un effet positif sur la prolifération des cellules du derme et de l'épiderme.

Ainsi, l'invention a donc également pour autre objet, un procédé cosmétique non thérapeutique pour améliorer la fermeté de la peau, notamment en favorisant la régénération du derme et de l'épiderme de la peau, caractérisé en ce qu'il consiste à appliquer sur les zones de la peau concernées au moins une composition cosmétique telle que définie ci-dessus, c'est-à-dire une composition cosmétique topique contenant une quantité efficace de cellules végétales dédifférenciées et élicitées de Bougainvillier.

Enfin, l'invention a pour objet l'utilisation non thérapeutique d'une composition cosmétique à application topique comprenant des cellules végétales dédifférenciées et élicitées de Bougainvillier pour améliorer la fermeté de la peau, notamment en favorisant la régénération du derme et de l'épiderme de la peau.

L'utilisation cosmétique de la composition cosmétique conforme à l'invention comprend tous les soins du corps et de la peau y compris les produits solaires, protecteurs et bronzants, les produits anti-âge, anti-séborrhéiques, toniques, les produits assurant l'amélioration de l'aspect de la peau y compris le traitement acnéique et les rougeurs cutanées.

Les exemples suivants illustrent l'invention plus en détail sans en limiter la portée. Dans tous les exemples de compositions qui suivent, les parties sont exprimées en masse, sauf indication contraire.

### EXEMPLES

### EXEMPLE 1 : Préparation d'une suspension de cellules dédifférenciées et élicitées de Bougainvillier

### 1. Prélèvement et culture des cellules dédifférenciées de Bougainvillier

Des morceaux de feuilles d'environ 5 à 10 mm ont été prélevés sur des feuilles fraîches de bougainvillier (*Bougainvillea glabra*).

Les morceaux de feuilles ont ensuite été stérilisés, puis mis en culture dans des boites stériles à température ambiante sur un milieu nutritif synthétique (milieu de Murashige et Skoog gélosé), jusqu'à formation, au niveau des explants, d'amas cellulaire dédifférenciées, appelés cals ou cellules mères.

Les cellules mères ont été prélevées au bout de 30 jours, puis repiquées sur un milieu nutritif neuf dans les mêmes conditions de culture que précédemment jusqu'à obtention de cals friables.

On a ainsi obtenu une collection de souches dédifférenciées stables présentant des caractéristiques de croissance et de production de métabolites.

### 2. Elicitation des cellules obtenues ci-dessus à l'étape précédente

L'élicitation des cellules a été réalisée par irradiation par la lumière UV à 180 nm pendant 2 heures, à l'aide d'une lampe Wilbert-Lourmat T-30C (600 µW/m²), positionnée à une distance de 1 m en éclairage direct au-dessus des cellules.

### 3. Préparation de la suspension cellulaire

Après l'élicitation, un morceau de cal a été prélevé puis mis en suspension dans un milieu nutritif liquide (milieu de Murashige et Skoog sans gélose).

A la fin de la culture (15 jours), les cellules ont été filtrées pour éliminer le milieu de culture puis rincées à l'eau froide (4°C). On a ainsi obtenu une biomasse fraîche d'environ 250 g de cellules par litre de culture.

Les cellules ont ensuite été mises en suspension dans de la glycérine végétale, à raison de 20 g de cellules pour 80 g de glycérine végétale, soit 20 % en masse.

On a obtenu une suspension de couleur beige-marron.

### EXEMPLE 2 : Mise en évidence de l'absence de cytotoxicité de la suspension de cellules végétales de Bougainvillier conforme à l'invention

Dans cet exemple on a testé la cytotoxicité éventuelle de la suspension de cellules dédifférenciées de *Bougainvillea glabra* telle qu'obtenue ci-dessus par le procédé de l'exemple 1.

L'essai a été réalisé *in vitro* sur épidermes humains reconstruits (« *Reconstructed Human Epidermis* », RHE) modèle RHEps, de surface 0,5 cm², vendus par la société SkinEthic ®.

La suspension glycérinée de cellules obtenue ci-dessus a été testée après dilution à 0,5 % dans avec du milieu de culture liquide (milieu Murashige et Skoog non gélosé) en application topique pendant 24 heures sur les épidermes, à raison de 2 µl par cm².

Les images histologiques (non représentées), obtenues après coloration à l'hématéine/éosine/safran (HES), des épidermes traités par la suspension étaient comparables à celles des épidermes témoins n'ayant reçu aucun traitement.

Ces résultats sont significatifs d'une absence de toxicité cutanée.

### EXEMPLE 3 : Mise en évidence de l'incorporation des cellules dédifférenciées et élicitées de Bougainvillier dans les différentes couches de l'épiderme

Dans cet exemple on a étudié l'aptitude des cellules dédifférenciées et élicitées de Bougainvillier à pénétrer dans les différentes couches de l'épiderme. En effet, les cellules végétales cultivées *in vitro,* sont constituées d'une membrane bi-lipidique, sont les constituants sont identiques aux lipides inter-cornéocytaires de la couche supérieure de l'épiderme. Cette similitude structurale confère aux cellules végétales une grande affinité pour la peau.

### 3.1. Principe du test

Le principe du test est basé l'utilisation de la protéine verte fluorescente (connue également sous la dénomination anglophone « Green Fluorescent Protein » ou GFP), afin de visualiser le passage des cellules de Bougainvillier dans les différentes couches de l'épiderme.

### 3.2. Protocole

5 mM de GFP ont été ajoutés à la suspension de cellules végétales de *Bougainvillea glabra* préparée ci-dessus à l'exemple 1. Le mélange a été incubé pendant 4 heures à 37°C dans une atmosphère contenant 5 % de CO₂.

Une composition à 5 mM de GFP dans du milieu de culture Murashige et Skoog non gélosé a également été préparée.

L'étude a été réalisée sur des épidermes humains reconstruits *in vitro,* de modèle RHEps, fabriqués par la société SkinEthic. Ils ont été répartis en 3 lots (n=4, c'est-à-dire 4 épidermes par lot) :
**Lot 1** : 4 épidermes non traités ;
**Lot 2** : 4 épidermes traités par la GFP seule ;
**Lot 3** : 4 épidermes traités avec des cellules végétales dédifférenciées et élicitées de Bougainvillier incubées avec la GFP.

A réception, les épidermes ont été transférés dans des plaques de 12 puits contenant du milieu de culture pour épiderme vendu par la société SkinEthic (0,5 ml/puits) et placés à l'incubateur à 37°C, dans une atmosphère à 5% de CO₂ saturée d'humidité, pendant 24 heures avant de procéder au traitement des tissus.

2 µl/cm² de chaque échantillon à tester (Solution de GFP seule ou Suspension de cellules incubées avec la GFP) ont été appliqués en topique sur les épidermes (surface des épidermes : 0,5 cm²). Après traitement, les épidermes ont été replacés à l'étuve à 37°C et incubés pendant 2 heures (2 épidermes par lot) ou pendant 12 heures (2 épidermes par lot) en atmosphère air/CO₂ (95%/5% : v/v).

Après incubation, les épidermes ont été fixés dans une solution de formaldéhyde à 10 % puis inclus dans des blocs de paraffine. Des coupes verticales de 4 µm ont été réalisées au microtome, puis observées et photographiées sous un microscope optique de fluorescence (Zeiss).

### 3.3. Résultats

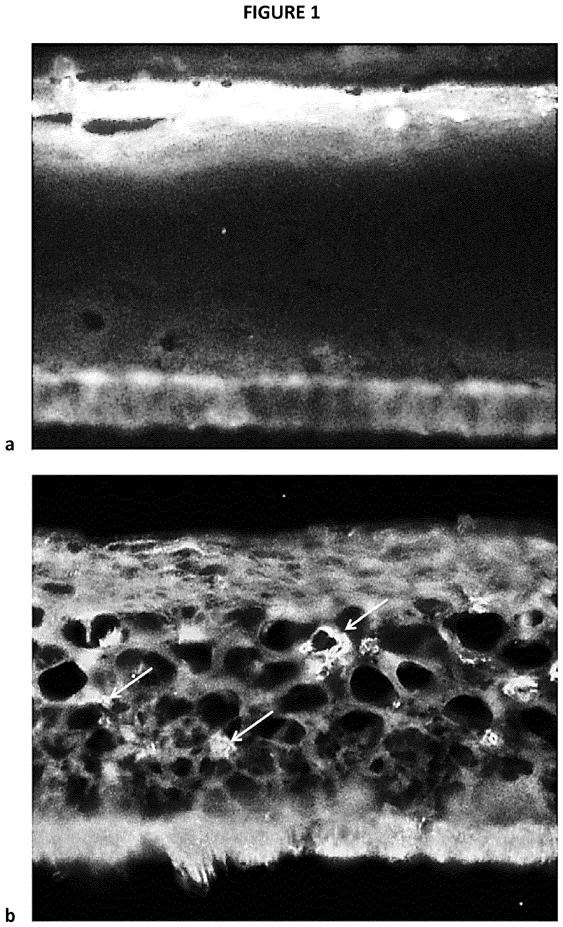

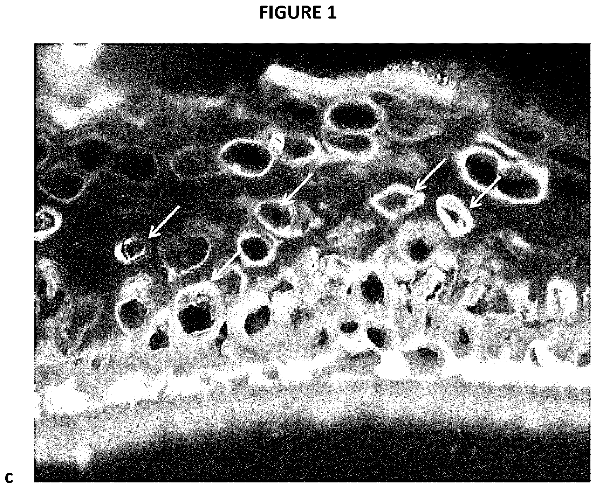

Les résultats obtenus sont reportés sur la figure 1 annexée qui donne une photographie d'un épiderme traité par la solution de GFP seule après 12 heures d'incubation (figure la), la photographie d'un épiderme traité pendant 2 heures avec la suspension de cellules dédifférenciées et élicitées de Bougainvillier préalablement incubées avec la GFP (figure 1b), ainsi que la photographie d'un épiderme traité pendant 12 heures avec la suspension de cellules dédifférenciées et élicitées de Bougainvillier préalablement incubées avec la GFP (figure 1c). Sur ces figures, les flèches blanches indiquent les cellules de Bougainvillier rendues fluorescence par la GFP et leur incorporation dans la couche de l'épiderme.

Ces résultats montrent que le traitement des épidermes par la solution de GFP seule n'induit aucun passage de cette dernière, aucun marquage fluorescent n'étant observé au sein des différentes couches de l'épiderme, la majorité de la fluorescence étant concentrée dans la couche cornée (figure la).

Par contre, concernant les épidermes traités avec la suspension de cellules dédifférenciées et élicitées de Bougainvillier préalablement incubées avec la GFP pendant 2 heures (figure 1b), on observe un passage de la protéine. En effet, un marquage fluorescent est observé au sein de l'épiderme, ce qui témoigne de la présence de la GFP et par conséquent de l'incorporation des cellules végétales dans les différentes couches de l'épiderme (couche granuleuse et couche spineuse).

Le traitement des épidermes pendant 12 heures avec la suspension de cellules dédifférenciées et élicitées de Bougainvillier préalablement incubées avec la GFP (figure 1c) conduit également à un marquage fluorescent, celui-ci étant observé au sein de toutes les couches, ce qui est significatif de l'incorporation desdites cellules aussi bien au niveau des couches granuleuse et spineuse que de la couche basale.

Ainsi, cet exemple démontre que les cellules dédifférenciées et élicitées de Bougainvillier préparées selon l'invention peuvent pénétrer au travers des différentes couches de l'épiderme pour atteindre la couche basale.

### EXEMPLE 4 : Evaluation de l'effet des cellules dédifférenciées et élicitées de Bougainvillier sur la régénération de l'épiderme

### 4.1 Principe du test

Le test est basé sur l'évaluation immunohistologique de l'index de prolifération Ki-67 afin d'estimer l'effet des cellules dédifférenciées et élicitées de Bougainvillier sur le potentiel de renouvellement cellulaire de la lame basale de l'épiderme.

En effet, l'antigène Ki-67 fait partie des marqueurs de prolifération. Cet antigène est présent sur une protéine nucléaire de 360 kDa (numéro d'accession P46013) présent dans les cellules prolifératives. L'antigène Ki-67 fut décrit par Gerdes J *et al,* en 1983 après immunisation des souris par injection de noyaux de cellules de lymphome provenant d'un lymphome de Hodgkin (Gerdes J. et al, International Journal of Cancer, 1983, Volume 31, Issue 1, pages 13-20).

Il est présent au niveau du noyau des cellules prolifératives, en phases G₁, S, G₂ et M et absent sur les cellules en cycle de repos (phase G₀). Sa fonction précise n'est pas connue, cependant une participation au maintien du pouvoir prolifératif ou au contrôle du cycle cellulaire est suggérée.

L'antigène Ki-67 peut être détecté par un anticorps Ki-67, à savoir l'anticorps monoclonal MIB-1 (société R&D Systems) en immunohistochimie. En pratique, l'index de marquage par le Ki-67 représente le pourcentage de noyaux colorés par l'anticorps MI1-B.

### 4.2 Protocole

L'étude a été réalisée sur des épidermes humains reconstruits *in vitro,* de modèle RHEps, fabriqués par la société SkinEthic. Ils ont été répartis en 2 lots (n= 4, c'est-à-dire 4 épidermes par lot) :

### Lot 1 : 4 épidermes non traités ;

**Lot 2 :** 4 épidermes traités avec la suspension de cellules végétales dédifférenciées et élicitées de Bougainvillier préparée ci-dessus à l'exemple 1, après dilution à 0,5 % dans avec du milieu de culture Murashige et Skoog non gélosé, à raison de 2 µl/cm², à 37°C, sous une atmosphère à 5% CO₂ pendant 24 heures.

Les épidermes du lot 1, ainsi que les épidermes du lot 2 traités par les cellules végétales dédifférenciées et élicitées de Bougainvilliers ont ensuite été fixés dans une solution de formaldéhyde à 10 %, puis inclus dans des blocs de paraffine. Des coupes verticales de 5 µm ont été réalisées au microtome. Chacune des coupes a ensuite été mise en contact avec une solution d'anticorps monoclonal MI1-B selon le protocole indiqué par le fournisseur R&D Systems. La révélation a été faite par la méthode peroxydase-anti peroxydase après démasquage antigénique par prétraitement à la chaleur également selon le protocole indiqué par R&D Systems. Le marquage par chromogène DAB (diamino-3,3'-benzidine tétrachlorhydrate) révèle en brun les sites nucléaires KI-67 de la fraction de cellules en croissance exprimées en phases G₁ et S (Phase de latence et synthèse de la cellule) ; G₂ (Phase de dédoublement des constituants cellulaire) et M (mitose).

L'index de prolifération KI-67 des épidermes traités par les cellules végétales dédifférenciées et élicitées de Bougainvillier a été évalué par comptage des sites nucléaires colorés, à raison de 10 champs par lame, au microscope optique, grossissement x 250, comparativement aux coupes des épidermes témoins non traités. Les résultats obtenus sont présentés dans le tableau 1 ci-après :

**TABLEAU 1**

| | **Nombre de cellules colorées** | **Différence par rapport au témoin (%)** |
|---|---|---|
| **Epidermes Témoins** | 16,5 ± 3 | - |
| **Epidermes traités par les cellules de Bougainvillier** | 20,8 ± 0,8 ⁽¹⁾ | + 26 |

| | | |
|---|---|---|
| ¹ : Significativement différent par rapport au témoin, p<0,05 (« *Wilcoxon Rank Sum Test* ») | | |

Ces résultats montrent que le traitement des épidermes par les cellules végétales dédifférenciées et élicitées de Bougainvillier a induit une augmentation de la prolifération cellulaire et par conséquent a un effet positif sur la régénération de l'épiderme.

### EXEMPLE 5 : Sérum pour le soin de la peau

Par les techniques usuelles, on a préparé un sérum pour le soin de la peau ayant la composition massique indiquée ci-après.

| | |
|---|---|
| - Glutamate diacétate tetrasodique | 0,07 g |
| - Butylèneglycol d'origine végétale vendu sous la dénomination commerciale Zemea ® par la société DKSH | 5,00 g |
| - Gomme de xanthane | 0,07 g |
| - Hydroxyéthyl cellulose | 0,30 g |
| - Lécithine hydrogénée | 1,00 g |
| - Triglycérides d'acides caprique et caprylique vendus sous la dénomination commerciale Myritol ® 318 par la société BASF | 3,00 g |
| - Stéaroyl-2-lactylate de sodium | 0,20 g |
| - Glycéryl undécylénate | 0,50 g |
| - Tetraisopalmitate d'ascorbyle | 0,10 g |
| - Sel de sodium de l'acide dihydroacétique | 0,10 g |
| - Hyaluronate de sodium | 0,15 g |
| - Tréhalose | 1,00 g |
| - Fucose | 0,50 g |
| - Suspension de cellules végétales telle que préparée à l'exemple 1 ci-dessus | 2,00 g |
| - Amidon de tapioca (manioc) | 0,35 g |
| - Parfum | 0,10 g |
| - Eau déminéralisée qsp | 100,00 g |

Ce sérum est destiné à raffermir et régénérer la peau et peut être utilisé une à deux fois par jour.

### EXEMPLE 6 : Crème anti-âge

Par les techniques usuelles, on a préparé une crème anti-âge ayant la composition massique suivante :

| | |
|---|---|
| - Glutamate diacétate tetrasodique | 0,10 g |
| - Butylèneglycol d'origine végétale vendu sous la dénomination commerciale Zemea ® par la société DKSH | 5,00 g |
| - Glycérine vendue sous la dénomination commerciale Glycérine Bio par la société IES | 8,00 g |
| - Bétaïne | 2,00 g |
| - Hydroxyéthyl cellulose | 0,50 g |
| - Déhydroacétate de sodium | 0,15 g |
| - Emulsionnant lamellaire naturel composé de phospholipides et de lipides végétaux, vendu sous la dénomination commerciale Biophilic ® H par la société Lucas Meyer Cosmetics | 4,00 g |
| - Glycéryl stéarate citrate | 0,80 g |
| - Squalane d'origine végétale vendu sous la dénomination commerciale Phytosqualane par la société Sophim | 5,00 g |
| - Triglycérides d'acides caprique et caprylique vendus sous la dénomination commerciale Myritol ® 318 par la société BASF | 7,00 g |
| - Alcool butylique | 1,00 g |
| - Alcool béhénylique | 0,75 g |
| - Huile de Macadamia | 2,00 g |
| - Huile de karité oléique vendue sous la dénomination commerciale Beurre de *Vitellaria nilotica* par la société Olvea | 4,00 g |
| - Glycéryl undécylénate | 0,50 g |
| - Tetraisopalmitate d'ascorbyle | 0,25 g |
| - Suspension de cellules végétales telle que préparée à l'exemple 1 ci-dessus | 5,00 g |
| - Géranyl géranyl propanol vendu sous la dénomination commerciale Juvinity® par la société Sederma | 2,00 g |
| - Parfum | 0,25 g |
| - Hyaluronate de sodium | 0,20 g |
| - Tréhalose | 1,00 g |
| - Mélange de palmitoyl oligopeptide et de palmitoyl-tetrapeptide-7 vendu sous la dénomination commerciale Matrixyl ® 3000 par la société Sederma Corp. | 3,00 g |
| - Eau déminéralisée qsp | 100,00 g |

Cette crème anti-âge peut être utilisée une à deux fois par jour par application sur les zones de la peau à traiter.

### EXEMPLE 7 : Lotion tonique rafraîchissante

Par les techniques usuelles, on a préparé une lotion tonique rafraichissante ayant la composition massique suivante :

| | |
|---|---|
| - Butylèneglycol d'origine végétale vendu sous la Dénomination commerciale Zemea ® par la société DKSH | 5,00 g |
| - Acide galacturonique | 0,10 g |
| - Acide anisique | 0,10 g |
| - Mélange d'acide lévulinique et de lévulinate de sodium vendu sous la dénomination commerciale Dissolvine ® GL 38 par la société AkzoNobel | 0,30 g |
| - Sel de sodium de l'acide pyrrolidone carboxylique (Sodium PCA) | 0,50 g |
| - Eau de menthe poivrée (société Herbarom) | 10,00 g |
| - Eau de Mélisse (société Herbarom) | 10,00 g |
| - Eau de Tilleul (société Herbarom) | 10,00 g |
| - Eau de Rosa Centifolia (société Herbarom) | 25,00 g |
| - Extrait de fleurs de Souci | 1,00 g |
| - Suspension de cellules végétales telle que préparée à l'exemple 1 ci-dessus | 1,00 g |
| - Eau de Romarin (société Herbarom) | 3,00 g |
| - Eau déminéralisée qsp | 100,00 g |

Cette lotion tonique rafraichissante peut être utilisée une à deux fois par jour par application sur les zones de la peau à traiter.

## Revendications

1. Utilisation de cellules végétales dédifférenciées et élicitées de Bougainvillier à titre d'ingrédient actif dans une composition cosmétique.

2. Utilisation selon la revendication 1, **caractérisée en ce que** lesdites cellules de Bougainvillier sont sous la forme d'une suspension dans la glycérine.

3. Utilisation selon la revendication 2, **caractérisée en ce que** la quantité de cellules dédifférenciées et élicitées de Bougainvillier au sein de ladite suspension varie de 20 à 30 % en masse par rapport à la masse totale de ladite suspension.

4. Composition cosmétique à application topique **caractérisée en ce qu'**elle comprend, à titre d'ingrédient actif, des cellules végétales dédifférenciées et élicitées de Bougainvillier et au moins un support cosmétiquement acceptable.

5. Composition selon la revendication 4, **caractérisée en ce qu'**elle comprend de 0,001 à 10 % en masse d'une suspension de cellules végétales dédifférenciées et élicitées de Bougainvillier dans la glycérine.

6. Composition selon la revendication 4 ou 5, **caractérisée en ce qu'**elle contient un ou plusieurs ingrédient actifs secondaires choisis parmi un agent anti-âge, l'acétyl tetrapeptide-5, un extrait de plancton, un extrait de maca, un extrait de pétales de coquelicot, une combinaison d'extraits d'anchuse, de coquelicot et de passiflore, un extrait de graines de mimosa, un extrait de pétales de souci, un extrait de barbitamao, un extrait de kigelia africana, un extrait d'avoine, la vitamine C, la vitamine E et l'hexylrésorcinol.

7. Composition selon l'une quelconque des revendications 4 à 6, **caractérisée en ce qu'**elle se présente sous forme de gel, d'émulsion, d'émulsion biphasique huile-dans-eau ou eau-dans-huile, d'huile corporelle, de masque, de pommade, d'onguent, de lotion ou de solution concentrée.

8. Composition selon l'une quelconque des revendications 4 à 7, **caractérisée en ce qu'**elle comprend en outre des agents de protection contre les rayons ultraviolets ou des pigments formant écran anti-ultraviolet.

9. Procédé cosmétique non thérapeutique pour améliorer la fermeté de la peau, **caractérisé en ce qu'**il consiste à appliquer sur les zones de la peau concernées au moins une composition cosmétique telle que définie à l'une quelconque des revendications 4 à 8.

10. Utilisation non thérapeutique d'une composition cosmétique à application topique comprenant des cellules végétales dédifférenciées et élicitées de Bougainvillier pour améliorer la fermeté de la peau.

## Patentansprüche

1. Verwendung entdifferenzierter Pflanzenzellen aus der Bougainvillea als Wirkstoff einer kosmetischen Zusammensetzung.

2. Verwendung nach Anspruch 1, **dadurch gekennzeichnet, dass** die Bougainvillea-Zellen in Form einer Suspension in Glycerin vorliegen.

3. Verwendung nach Anspruch 2, **dadurch gekennzeichnet, dass** die Menge der entdifferenzierten Bougainvillea-Zellen in der Suspension, bezogen auf die Gesamtmasse der Suspension, zwischen 20 und 30 Masse-% liegt.

4. Kosmetische Zusammensetzung zur topischen Anwendung, **dadurch gekennzeichnet, dass** diese als Wirkstoff entdifferenzierte Pflanzenzellen aus der Bougainvillea sowie mindestens einen kosmetisch unbedenklichen Träger umfasst.

5. Zusammensetzung nach Anspruch 4, **dadurch gekennzeichnet, dass** sie zu 0,001 - 10 Masse-% eine Suspension entdifferenzierter Pflanzenzellen aus der Bougainvillea in Glycerin umfasst.

6. Zusammensetzung nach Anspruch 4 oder 5, **dadurch gekennzeichnet, dass** sie mindestens einen Nebenwirkstoff umfasst, der aus der nachfolgenden Gruppe gewählt ist: ein Anti-Aging-Mittel, Acetyl-Tetrapeptid-5, ein Plankton-Extrakt, ein Maca-Extrakt, ein Mohnblütenextrakt, eine Kombination aus Ochsenzungen-, Mohn- und Passionsblumenextrakt, ein Mimosensamenextrakt, ein Ringelblumenblütenextrakt, ein Barbitamao-Extrakt, ein Kigelia africana-Extrakt, ein Haferextrakt, Vitamin C, Vitamin E und Hexylresorcin.

7. Zusammensetzung nach einem der Ansprüche 4 - 6, **dadurch gekennzeichnet, dass** sie in Form eines Gels, einer Emulsion, einer zweiphasigen Öl-Wasser- oder Wasser-Öl-Emulsion, eines Hautöls, einer Maske, einer Salbe, einer Lotion oder einer konzentrierten Lösung vorliegt.

8. Zusammensetzung nach einem der Ansprüche 4 - 7, **dadurch gekennzeichnet, dass** sie ferner UV-Schutzmittel oder Pigmente, die eine Anti-UV-Abschirmung bilden, umfasst.

9. Nicht therapeutisches kosmetisches Verfahren zur Verbesserung der Festigkeit der Haut, **dadurch gekennzeichnet, dass** es darin besteht, auf die betroffenen Hautbereiche mindestens eine kosmetische Zusammensetzung nach einem der Ansprüche 4 - 8 anzuwenden.

10. Nicht therapeutische Verwendung einer kosmetischen Zusammensetzung zur topischen Anwendung, umfassend entdifferenzierte Pflanzenzellen aus der Bougainvillea, um die Festigkeit der Haut zu verbessern.

## Claims

1. Use of dedifferentiated and elicited plant cells of Bougainvillaea as active ingredient in a cosmetic composition.

2. The use according to claim 1, **characterized in that** said Bougainvillaea cells are in the form of a suspension in glycerine.

3. The use according to claim 2, **characterized in that** the amount of dedifferentiated and elicited Bougainvillaea cells in said suspension varies from 20 to 30 weight % relative to the total weight of said suspension.

4. Cosmetic composition for topical application, **characterized in that**, as active ingredient, it comprises dedifferentiated and elicited plant cells of Bougainvillaea and at least one cosmetically acceptable carrier.

5. The composition according to claim 4, **characterized in that** it comprises from 0.001 to 10 weight % of a suspension of dedifferentiated and elicited plant cells of Bougainvillaea in glycerine.

6. The composition according to claim 4 or 5, **characterized in that** it contains one or more secondary active ingredients selected from among an anti-aging agent, acetyl-tetrapeptide-5, a plankton extract, maca extract, poppy petal extract, a combination of Anchusa, poppy and Passiflora extracts, an extract of mimosa seeds, marigold petal extract, barbitamao extract, Kigelia africana extract, oat extract, vitamin C, vitamin E and hexylresorcinol.

7. The composition according to any of claims 4 to 6, **characterized in that** it is in the form of a gel, emulsion, biphase oil-in-water or water-in-oil emulsion, body oil, mask, ointment, unguent, lotion or concentrated solution.

8. The composition according to any of claims 4 to 7, **characterized in that** it further comprises protective agents against ultraviolet radiation or pigments forming an anti-ultraviolet screen.

9. Non-therapeutic cosmetic method to improve the skin firmness, **characterized in that** it consists of applying to the skin areas concerned at least one cosmetic composition such as defined in any of claims 4 to 8.

10. Non-therapeutic use of a cosmetic composition for topical application comprising dedifferentiated and elicited plant cells of Bougainvillaea to improve skin firmness.
